# EUROPEAN PATENT SPECIFICATION

(11) **EP 1 981 343 B1**
(45) Date of publication and mention of the grant of the patent: **13.05.2015**
(21) Application number: 07716706.2
(22) Date of filing: 17.01.2007
(51) Int. Cl.: A01N 43/78, A61K 31/425

(54) **TREATMENT OF INFLAMMATORY DISORDERS WITH TRIAZOLE COMPOUNDS**
BEHANDLUNG VON ENTZÜNDUNGSERKRANKUNGEN MIT TRIAZOLVERBINDUNGEN
TRAITEMENT DE TROUBLES INFLAMMATOIRES AVEC DES COMPOSES DE TRIAZOLE

(30) Priority: 17.01.2006 US 759027 P
(43) Date of publication of application: 22.10.2008
(73) Proprietor: Stiefel Laboratories, Inc., Coral Gables, FL 33134 (US)
(72) Inventor: COOLS, Marina, B-2470 Retie (BE); BORGERS, Marcel, B-2440 Geel (BE)
(74) Representative: Reeves, Julie Frances
(86) International application number: PCT/US2007/001202
(87) International publication number: WO 2007/084542

(56) References cited:
- WO-A1-96/30350
- WO-A1-2005/019216
- WO-A1-2005/044287
- WO-A2-2006/031929
- US-A- 6 124 330
- US-B1- 6 787 556
- N. E. MEALY, M. BAYES: "Annual update 2004/2005 - Treatment of dermatological disorders" DRUGS OF THE FUTURE, vol. 30, no. 8, 2005, pages 815-869, XP002605929

## Description

### Field of the Invention

The present invention relates to a triazole compound for use in a method of reducing or preventing inflammation in a mammal.

### Background of the Invention

Inflammation is the first response of the body to injury, irritation or disease. It is characterized in the acute form by the classical signs of pain, heat, redness, swelling and loss of function. Irritant dermatitis, for example, is an inflammation of the skin caused by direct contact with an irritating substance and is characterized by increased blood flow, redness and transepidermal water loss (TEWL).

Prostaglandins play a major role in the inflammation process, and the inhibition of prostaglandin production, especially production of PGG₂, PGH₂ and PGE₂, has been a common target of antiinflammatory drug discovery. However, common non-steroidal antiinflammatory drugs (NSAIDs) that are active in reducing the prostaglandin-induced pain and swelling associated with the inflammation process, are also active in affecting other prostaglandin-regulated processes not associated with the inflammation process. Thus, use of high doses of some of the most common NSAIDs can produce severe side effects, such as life-threatening ulcers, that limit their therapeutic potential. An alternative to NSAIDs is the use of corticosteroids, which have even more drastic side effects, especially when long-term therapy is involved.

Tumor necrosis factor (TNF), interleukin-12 (IL-12) and interleukin-1 (IL-1) are important biological entities collectively referred to as pro-inflammatory cytokines. These, along with several other related molecules, mediate the inflammatory response associated with the immunological recognition of infectious agents. The inflammatory response plays an important role in limiting and controlling pathogenic infections.

Elevated levels of pro-inflammatory cytokines are also associated with a number of autoimmune-associated diseases such as toxic shock syndrome, rheumatoid arthritis, osteoarthritis, diabetes and inflammatory bowel disease *(*Dinarello et al., Rev. Infect. Disease 6, 51 (1984)). Typically, chronic elevation of inflammation causes or exacerbates much of the observed symptoms in these diseases. For example, inflammatory cells attack rheumatoid synovial tissue, resulting in destruction to cartilage and bone *(*Koch et al., J. Invest. Med. 43, 28-38 (1995)). Reduction of pro-inflammatory cytokines such as TNF (also referred to in its secreted cell-free form as TFNα) and IL-1β represents an accepted therapeutic approach for potential drug intervention in these diseases. A number of anti-cytokine therapies are currently in clinical trials. Efficacy has been demonstrated with a monoclonal antibody directed against TFNα in a number of autoimmune diseases, such as rheumatoid arthritis, Crohn's disease and ulcerative colitis (Rankin, E.C.C., et al., British J. Rheum. 35, 334-342 (1997) and Stack et al., Lancet 349, 521-524 (1997)).

IL-1 has been implicated as an immunological effector molecule in a large number of disease processes. Efficacy of a IL-1 receptor antagonist has been demonstrated for the treatment of rheumatoid arthritis (Antril, Amgen). It has been shown in human clinical trials that IL-1 receptor antagonists reduced the mortality rate in patients with septic shock syndrome (Dinarello, Nutrition 11, 492 (1995)). IL-1 has been detected in synovial fluid and in the cartilage matrix of osteoarthritic joints. Antagonists of IL-1 have been shown to diminish the degradation of cartilage matrix components in a variety of experimental models of arthritis (Chevalier, Biomed Pharmacother. 51, 58 (1997)).

Human interferon-inducible protein 10 (IP-10) is a member of the CXC subfamily of chemokines and attracts T-lymphocytes and natural killer cells. IP-10 is upregulated in psoriasis. In particular, epidermal keratinocytes of psoriatic lesions express elevated levels of IP-10. Suppression of IP-10 expression and secretion by activated keratinocytes may represent a novel target for therapeutic intervention of inflammatory skin disorders.

IL-12 is a pro-inflammatory cytokine, produced by immune effector cells such as activated monocytes/macrophages and dendritic cells and by keratinocytes, that fulfills a key role in the induction and maintenance of a variety of type 1 T helper cell (Th1)-mediated skin diseases. Therefore, its suppression in activated monocytes represents a novel target for therapeutic intervention.

COX-2 expression has been shown to be increased by cytokines and it is believed to be the isoform of cyclooxygenase responsible for inflammation (O'Banion et al., Proc. Natl. Acad. Sci. U.S.A., 89, 4888 (1992)). Accordingly, inhibitors of cytokines such as IL-1 would be expected to exhibit efficacy against those disorders currently treated with COX inhibitors such as the familiar non-steroidal antiinflammatory drugs (NSAIDs). These disorders include acute and chronic pain as well as symptoms of inflammation and cardiovascular disease.

Elevation of several cytokines has been demonstrated during active inflammatory bowel disease (IBD). A mucosal imbalance of intestinal IL-1 and IL-1 receptor antagonists has been shown to be present in patients with IBD. Insufficient production of endogenous IL-1 receptor antagonists may contribute to the pathogenesis of IBD (Cominelli et al., Aliment. Pharmacol. Ther. 10, 49 (1996)). IL-1 receptor antagonists show a clear relationship to acute inflammatory events as well as to the different disease stages in the pathophysiology of HIV infection *(*Kreuzer et al., Clin. Exp. Immunol. 109, 54 (1997)).

Pro-inflammatory cytokines such as TFNα and IL-1α are also important mediators of septic shock and associated cardiopulmonary dysfunction, acute respiratory distress syndrome (ARDS) and multiple organ failure. In a study of patients presenting at a hospital with sepsis, a correlation was found between TFNα and IL-6 levels and septic complications *(*Terregino et al., Ann. Emerg. Med. 35, 26 (2000)).

IL-1 has also been shown to induce uveitis in rats which could be inhibited with IL-1 blockers (Xuan et al., J. Ocular Pharmacol. and Ther., 14, 31 (1998)). IL-1 has been shown to be essential for the development of both irritant and allergic contact dermatitis. Epicutaneous sensitization can be prevented by administration of an anti-IL-1 monoclonal antibody before epicutaneous application of an allergen (Muller et al., Am. J. Contact Dermat. 7, 177 (1996)).

Triazole compounds have been used as therapeutic agents. See, e.g., U.S. Patents 6,124,330; 6,265,425 and 6,486,187.

The present invention relates to a triazole compound for use in a methos of reducing or preventing inflammation in a mammal, including a human, comprising administering to said mammal an amount of a triazole compound capable of modulating the expression and/or activity of one or more cytokines, including chemokines. In some embodiments, the inflammatory-associated disorder is an inflammatory skin disorder. In some embodiments, the expression and/or activity is inhibited. In another embodiment, the cytokine is IL-1 and/or IL-12.

According to the invention, the triazole compound is selected from Formula I or a pharmaceutically acceptable salt thereof 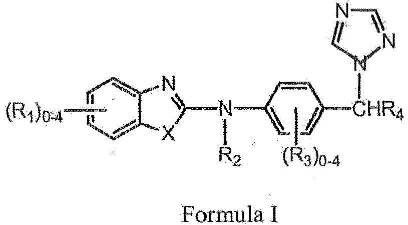 wherein
X is N, O or S; each R₁ and each R₃ is independently selected from the group consisting of C₁₋₈ alkyl, amino, alkylamino, halogen, hydroxy, alkoxy, aryl, aryloxy, -CF₃, -OCF₃, -CORₐ, -COORₐ, -CONRₐR_{b}, -NHCORₐR_{b}, -NHSO₂Rₐ, -SO₂Rₐ, -SO₃Rₐ or -SO₂NRₐR_{b}, wherein Rₐ and R_{b} are independently hydrogen, alkyl, cycloalkyl, aryl or aralkyl; R₂ is selected from the group consisting of H, C₁₋₆ alkyl, -CORₐ and -SO₂Rₐ; and R₄ is selected from the group consisting of C₁₋₈ alkyl, optionally substituted by at least one group selected from the group consisting of amino, alkylamino, halogen, hydroxy, alkoxy, -CF₃, -OCF₃, -CORₐ, -COORₐ and -CONRₐR_{b}.

In a preferred embodiment, the triazole of Formula I is rambazole, which has the following structure:

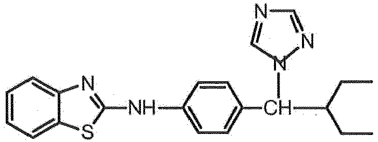

### Brief Description of the Drawings

The figures are for illustrative purposes and represent exemplary embodiments of present invention. Thus, the figures are not intended to limit the scope of the described invention.
**Figure 1** shows the effect of a topical gel formulation of rambazole (as an exemplary RAMBA) on the expression of the biomarker interleukin-la (IL-1) as compared to a placebo.
**Figure 2** shows the effect of rambazole on the expression of IL-1α as compared to the effect of retinoic acid on the expression of IL-1α.
**Figure 3** shows the effect of various concentrations of rambazole on cultures of monocytes activated with Zymosan or a 19 kDa lipoprotein. These results indicate the suppression of IL-12 by administration of rambazole.
**Figure 4** shows the effect of various concentrations of rambazole on IP-10 expression and secretion in IFNγ-activated normal human epidermal keratinocytes.
**Figure 5** shows the results of the frequency of inflammatory lesions during the course of a 12-week dosing of orally administered rambazole.
**Figure 6** shows the results of the frequency of non-inflammatory lesions during the course of a 16-week dosing of orally administered rambazole.
**Figure 7** shows the incidence of side effects related to cutaneous retinoic acid throughout a 16-week dosing of orally administered rambazole to subjects with moderate to severe facial acne vulgaris.
**Figure 8** shows the effect of topical rambazole on UVB in an investigator-blinded, placebo-controlled study.
**Figure 9** shows the effect of topical rambazole on patients suffering from mild to moderate acne vulguris.

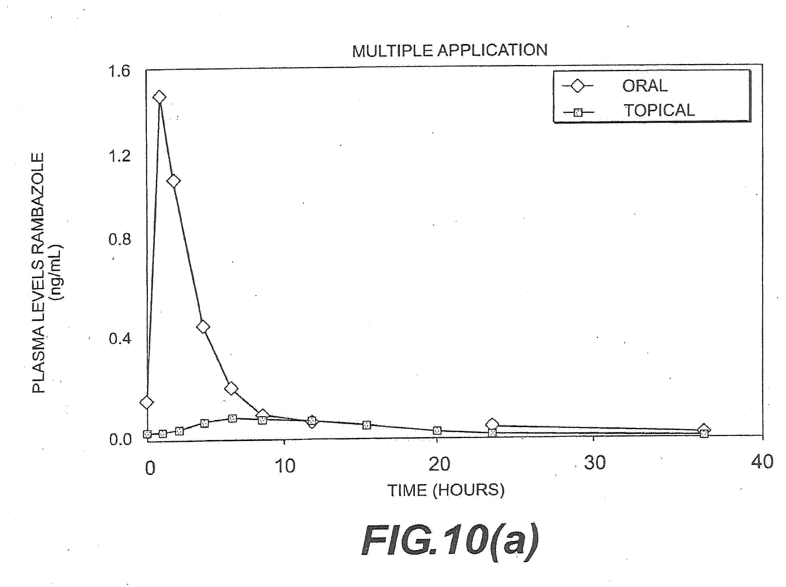

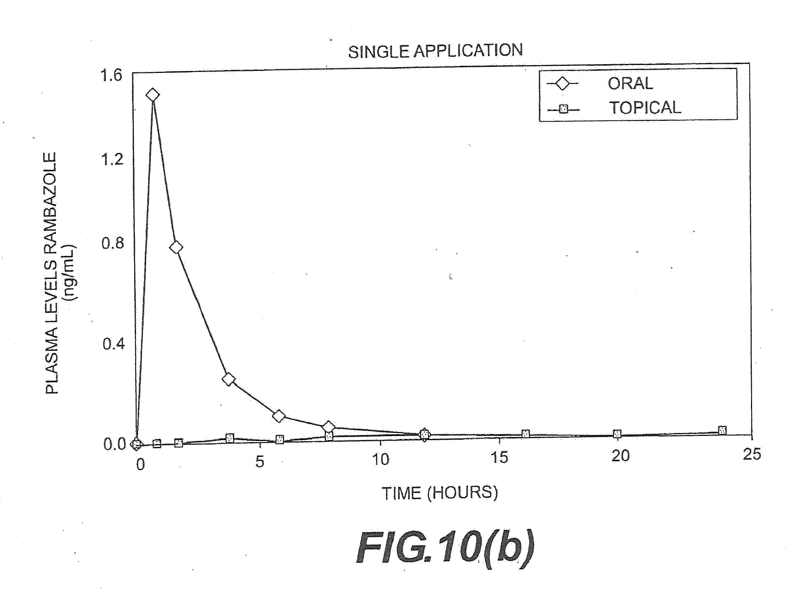

**Figure 10** shows the plasma levels of rambazole after single and multiple applications of topical rambazole in volunteers with acne vulguris and after oral dosing in healthy volunteers.

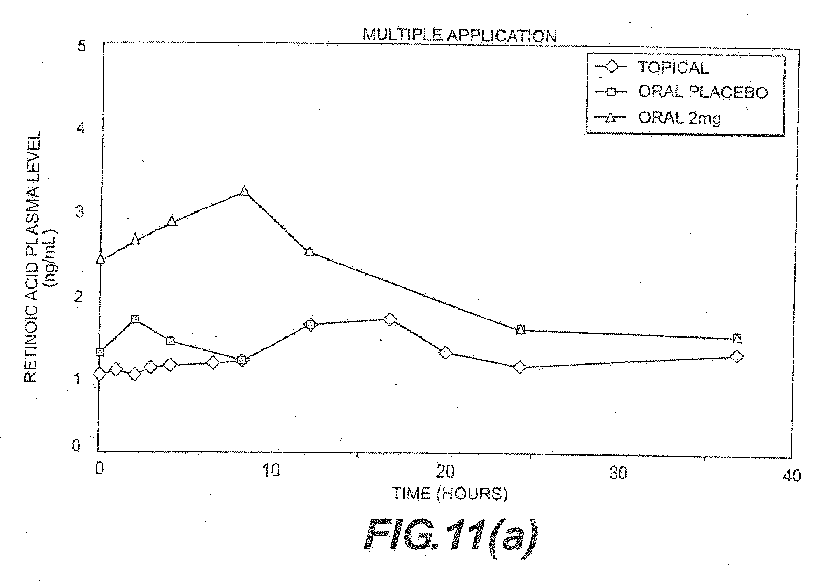

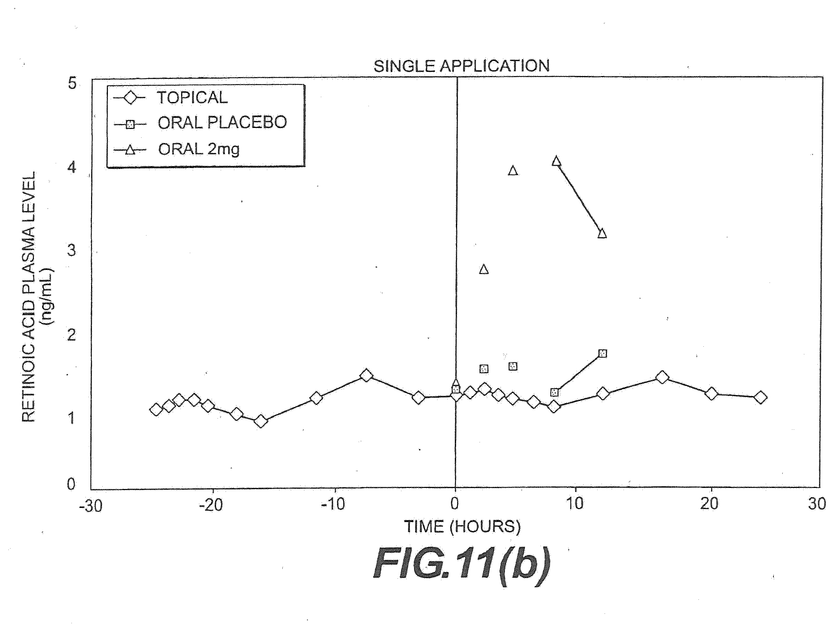

**Figure 11** shows retinoic acid plasma levels in patients suffering from acne vulguris after single and multiple applications of topical rambazole and in healthy volunteeers after oral dosing.
**Figure 12** shows the effect of rambazole on irritant dermatitis induced by sodium lauryl sulfate (SLS) as measured by visual scoring of redness (**12a**), chromametry (12b) and TEWL (**12c**).

### Detailed Description of the Invention

### Definitions

As defined herein, an "effective amount" means a sufficient but non-toxic amount of an active agent to provide the desired therapeutic effect. In an aspect of the invention, an effective amount includes the amount of a triazole compound of Formula I required to decrease cytokine and/or chemokine expression and/or activity such that a disease state associated with increased levels of cytokines and/or chemokines is treated. In another aspect of the invention, the targeted cytokine is IL-1 or IL-12.

As defined herein, "topical administration" means the delivery of a topical drug or pharmacologically active agent directly to the skin or mucosa of an individual.

As defined herein, "cytokine" means a non-antibody protein, secreted by inflammatory leukocytes and some non-leukocytic cells, that acts as an intercellular mediator of inflammation.

As defined herein, "chemokine" means a protein of the class of pro-inflammatory cytokines that have the ability to attract and activate leukocytes. They can be divided into at least three structural branches: c (chemokines, c), cc (chemokines, cc), and cxc (chemokines, cxc), according to variations in a shared cysteine motif.

As defined herein, "IL-1" (interleukin-1) means a soluble protein (17 kDa with 152 amino acids) secreted by monocytes, macrophages or accessory cells involved in the activation of both T-lymphocytes and B lymphocytes and potentiates their response to antigens or mitogens. Its biological effects include the ability to replace macrophage requirements for T-cell activation, as well as affecting a wide range of other cell types. At least two IL-1 genes are active and alpha and beta forms of IL-1 are recognized. It is released early in an immune system response by monocytes and macrophages and stimulates T-cell proliferation and protein synthesis.

As defined herein, "IL-12" (interleukin-12) is a 75 kD heterodimeric cytokine composed of disulfide-bonded 40 kD and 35 kD subunits that was originally identified by its ability to induce cytotoxic effector cells in synergy with less than optimal concentrations of interleukin-2. It is released by macrophages in response to infection and promotes the activation of cell-mediated immunity. Specifically, IL-12 triggers the maturation of Thl CD4 cells, specific cytotoxic T-lymphocyte responses and an increase in the activity of NK cells and consequently, it is the initiator of cell-mediated immunity. It enhances the lytic activity of NK cells, induces interferon production, stimulates the proliferation of activated T-tells and NK cells and is secreted by human B lymphoblastoid cells (NC 37). It may play a role in controlling immunoglobulin isotype selection as it also inhibits IgE synthesis (even in the presence of anti-IFN monoclonal antibody) and as a growth factor for activated CD4+ and CD8+ T-cells independently of interleukin-2, and for CD56+ NK cells but not resting peripheral blood mononuclear cells or resting or activated tonsillar B-cells. IL-12 was formerly referred to as cytotoxic lymphocyte maturation factor.

The invention is considered to include the enantiomeric, diastereomeric and tautomeric forms of all compounds of Formula I as well as their racemic mixtures. In addition, some of the compounds represented by Formula I are prodrugs, *i*.*e*., derivatives of an acting drug that possess superior delivery capabilities and therapeutic value as compared to the acting drug. Prodrugs are transformed into active drugs by *in vivo* enzymatic or chemical processes.

As defined herein, the term "alkyl" refers to both linear and branched chain radicals of up to 12 carbon atoms, unless otherwise indicated, and includes, but is not limited, to methyl, ethyl, propyl, isopropyl, butyl, isobutyl, sec-butyl, tert-butyl, pentyl, isopentyl, hexyl, isohexyl, heptyl, octyl, 2,2,4-trimethylpentyl, nonyl, decyl, undecyl and dodecyl. In an exemplary embodiment, "alkyl" is a C₁₋₆ group, which includes a one carbon unit (*i.e.,* methyl) up to a six-carbon unit (*i.e.,* hexyl). In another exemplary embodiment, "alkyl" is a C₁₋₈ group, which includes a one carbon unit (*i.e.,* methyl) up to a eight-carbon unit (*i.e.,* hexyl).

As defined herein, the term "amino" refers to NH₂.

As defined herein, the term "aminoalkyl" refers to at least one primary or secondary amino group bonded to any carbon atom along an alkyl chain.

As defined herein, "halogen" refers to fluorine, chlorine, bromine or iodine.

As defined herein, "hydroxy" refers to OH.

As defined herein, "alkoxy" refers to straight or branched chain radicals of up to 12 carbon atoms, unless otherwise indicated, bonded to an oxygen atom. Examples include methoxy, ethoxy, propoxy, isopropoxy and butoxy.

As defined herein, "aryl" refers to monocyclic or bicyclic aromatic ring systems containing from 6 to 12 carbons in the ring and optionally substituted with 1-3 substituents selected from alkoxy, alkyl, halogen and hydroxy. Examples include phenyl, biphenyl and napththalene.

As defined herein, the term "aryloxy" refers to an aryl group bonded to an oxygen atom. An example is phenoxy.

As defined herein, the term "aralkyl" refers to a C.sub.1-6 alkyl group containing an aryl substituent. Examples include benzyl, phenylethyl or 2-naphthylmethyl.

As defined herein, the term "cycloalkyl" refers to a ring composed of from 3 to 8 carbon atoms. Alkyl substituents may optionally be present on the ring. Examples include cyclopropyl, 1,1-dimethyl cyclobutyl, 1,2,3-trimethylcyclopentyl and cyclohexyl.

Unless otherwise specified, all other descriptions or listings of chemical functional groups are intended to have the meanings most commonly attributed to them by a person of ordinary skill in the art.

In an aspect of the invention, the triazole compounds of the invention may be used in combination with therapeutic azoles such as, but not limited to, ketoconazole, itraconazole, azoline, miconazole, metronidazole, liarozole, benzimidazole, benzothiazole, bifonazole, butaconazole nitrate, climbazole, clotrimazole, croconazole, eberconazole, econazole, elubiol, fenticonazole, fluconazole, flutimazole, isoconazole, lanoconazole, neticonazole, omoconazole, oxiconazole nitrate, sertaconazole, sulconazole nitrate, tioconazole, thiazole, terconazole and itraconazole.

Inflammation-associated disease states include, but are not limited to, those diseases associated with increased expression of a inflammatory cytokine and/or chemokine, for example, inflammatory skin disease, arthritis, toxic shock syndrome, diabetes and inflammatory bowel disease. Inflammatory skin diseases include, but are not limited to, atopic dermatitis, contact dermatitis, seborrheic dermatitis, rosacea, eczema, psoriasis, acne and urticaria. Psoriasis includes, but is not limited to, plaque psoriasis (psoriasis vulgaris or chronic plaque psoriasis). Arthritis includes, but is not limited to, rheumatoid arthritis, psoriatic arthritis, osteoarthritis, gout, lupus and fibromyalgia. Diabetes includes, but is not limited to, diabetes type II. Inflammatory bowel disease includes, but is not limited to, Crohn's disease.

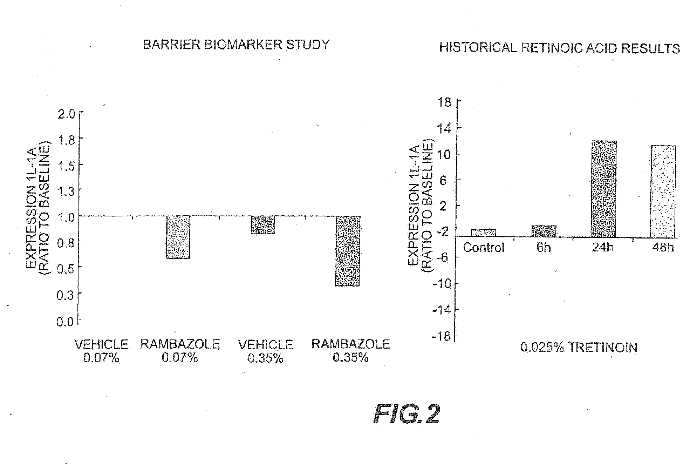

As indicated by Figure 2, rambazole was unexpectedly observed to decrease the expression of IL-1. In contrast, it has been previously shown that retinoic acid increases the expression of IL-1 in human epidermis (Bernard et al., Exp Dermatol. 11(1), 59-74 (2002)). Accordingly, administration of retinoic acid (in the form of the commercialized tretinoin-containing cream Retacnyl) and likely other retinoids result in increased inflammation while administration of the triazole compounds of the invention resulted in decreased inflammation. Also unexpectedly, rambazole-treated lesions (such as, for example, psoriatic lesions) continue to improve and show reduced inflammatory patterns for at least 3 months after discontinuation of treatment. Further, in contrast with retinoids such as isotretinoin, rambazole-treated inflammatory lesions responded rapidly within the first 4 weeks of treatment. This finding supports an antiinflammatory action for rambazole in addition to the typical effects on proliferation and differentiation.

The compositions and methods of the invention for therapeutic purposes can be utilized *in vivo,* ordinarily in mammals, such as humans, sheep, horses, cattle, pigs, dogs, cats, rats and mice or *ex vivo.* The invention is particularly useful in the treatment of human subjects.

Suitable pharmaceutical formulations for topical applications of the triazole compounds include, for example, ointments, salves, creams, gels, lotions, dressings, shampoos, tinctures, pastes and powders. Application of the compositions of the invention may also be by aerosol, *e.g.,* with a propellent such as nitrogen, carbon dioxide, a freon, or without a propellent such as a pump spray, drops, lotions, or a semisolid such as a thickened composition which can be applied by a swab. In addition, the triazole compositions may be applied as a transdermal patch.

Other suitable formulations include toilet waters, packs, skin milks or milky lotions. Such formulations often include therapeutically inactive components such as, for example, oils, fats, waxes, surfactants, humectants, thickening agents, antioxidants, viscosity stabilizers, chelating agents, buffers, preservatives, perfumes, dyestuffs, lower alcohols and the like. If desired, additional ingredients may be incorporated in the compositions of the invention such as antiinflammatory agents, antibacterials, antifungals, disinfectants, vitamins, sunscreens, antibiotics or anti-acne agents.

The pharmaceutical compositions of the invention may also optionally include other carriers, stabilizers, preservatives or adjuvants. For typical examples of these classes of compounds, see Remington: The Science and Practice of Pharmacy, Lippincott, Williams & Wilkins (2005), which is incorporated by reference in its entirety.

In an exemplary embodiment, the triazole compound is formulated as a gel. In another embodiment, the gel is an anhydrous gel. The gel may include an alcohol and a polyol. Suitable alcohols include, but are not limited to, methanol, ethanol, propanol, isopropanol, isobutanol or combinations thereof. Suitable polyols include, but are not limited to, polyethylene glycol, propylene glycol, ethylene glycol, a sugar or combinations thereof. The alcohol may be present in an amount of from about 5 to about 70 weight percent, such as from about 10 to about 50 weight percent. Similarly, the polyol may be present in an amount of from about 15 to about 80 weight percent, such as from about 25 to about 65 weight percent. In an exemplary embodiment, the triazole compound is formulated as an oral gel and not as a topical gel. In an exemplary embodiment, an oral gel formulation contains 0.20 mg or 0.5 mg or 0.75 mg or 1.0 mg or 1.5 mg or 2.0 mg of rambazole in combination with polyethylene glycol 400. In some embodiments, the oral gel formulation may be in the form of a soft gel capsule.

An effective amount of the triazole compound in the combination therapy composition or administered alone ranges from about 0.01 weight percent to about 25 weight percent. It may be appropriate to administer the triazole compound, either alone or in a combination therapy, once daily or as two, three, four or more sub-doses at appropriate intervals throughout the day. Sub-doses may be formulated as unit dosage forms, for example, containing 0.001 mg to 500 mg of active ingredient per unit dosage form.

The triazole compounds may also be administered orally. For oral administration, the triazoles can be formulated readily by combining them with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by combining the triazole compound with a solid excipient, optionally grinding a resulting mixture, and processing the mixture of granules, after adding suitable auxiliaries, if desired, to obtain tablets or dragee cores. Suitable excipients include, for example, fillers such as sugars, including lactose, sucrose, mannitol, or sorbitol; cellulose preparations such as maize starch, wheat starch, rice starch, potato starch, gelatin, gum tragacanth, methyl cellulose, hydroxypropylmethylcellulose, sodium carboxymethylcellulose and polyvinylpyrrolidone (PVP). If desired, disintegrating agents may be added, such as the cross-linked polyvinyl pyrrolidone, agar, alginic acid or a salt thereof, such as sodium alginate.

Pharmaceutically acceptable salts of the triazole compounds include the conventional non-toxic salts or the quaternary ammonium salts which are formed from inorganic or organic acids or bases. Examples of such acid addition salts include acetate, adipate, benzoate, benzenesulfonate, citrate, camphorate, dodecylsulfate, hydrochloride, hydrobromide, lactate, maleate, methanesulfonate, nitrate, oxalate, pivalate, propionate, succinate, sulfate and tartrate. Base salts include ammonium salts, alkali metal salts such as sodium and potassium salts, alkaline earth metal salts such as calcium and magnesium salts, salts with organic bases such as dicyclohexylamine salts and salts with amino acids such as arginine. Also, the basic nitrogen-containing groups may be quaternized with, for example, alkyl halides.

In the triazole compounds of Formula I, there may be zero R₁ groups or one R₁ group or two independently selected R₁ groups or three independently selected R₁ groups or four independently selected R₁ groups.

In the triazole compounds of Formula I, there may be zero R₃ groups or one R₃ group or two independently selected R₃ groups or three independently selected R₃ groups or four independently selected R₃ groups.

The exact dosage, frequency and duration of administration of the triazole compounds depend on the particular azole being used, the particular disease state being treated, the severity of the disease state being treated, the age, weight and general physical condition of the particular patient as well as other medication the patient may be taking, as is well known to those skilled in the art. Furthermore, it is evident that the effective frequency of administration of the combination therapy may be lowered or increased depending on the response of the treated patient and/or depending on the evaluation of the physician prescribing the therapeutic agent being used.

The triazole compounds may be used in combination with other therapeutic agents, such as, but not limited to, anti-inflammatory agents (*e.g.,* ibuprofen, fenoprofen, ketoprofen, naproxen, diclofenac, etodolac, meclofenamate sodium phenylbutazone, indomethacin, piroxicam, sulindac and tolmetin), anti-proliferating agents (*e.g.,* mycophenolate mofetil and evodiamine), anti-acne agents (*e.g.,* tretinoin, isotretinoin, salicylic acid, benzoyl peroxide and azelaic acid), anti-pruritic agents (*e.g.* azelastine, cetirizine permethrin and lindane), anti-aging agents and combinations thereof.

Without further description, it is believed that one of ordinary skill in the art can, using the preceding description and the following illustrative examples, make and utilize the compounds of the present invention and practice the claimed methods. The following working examples describe embodiments of the present invention, and are not to be construed as limiting in any way the remainder of the disclosure.

### Examples

### Example 1: Treatment of Psoriasis with Rambazole

Seventeen patients with a Psoriasis Area and Severity Index (PASI) score of at least 5 (moderate to severe plaque type psoriasis) received a daily oral dose of 1 mg of rambazole. The evolution of the PASI score was monitored after 1, 2, 4 and 8 weeks of treatment and at the end of a 2-week follow-up period. Overall, the mean reduction in PASI score for subjects that completed 8 weeks of treatment as 51.4%. The side effects tend to be mild to moderate and the treatment appears to be well tolerated. Biopsies from lesions were taken and surface markers were analyzed. See Table 2.

### Example 2: Treatment of Acne Vulgaris with Oral Rambazole

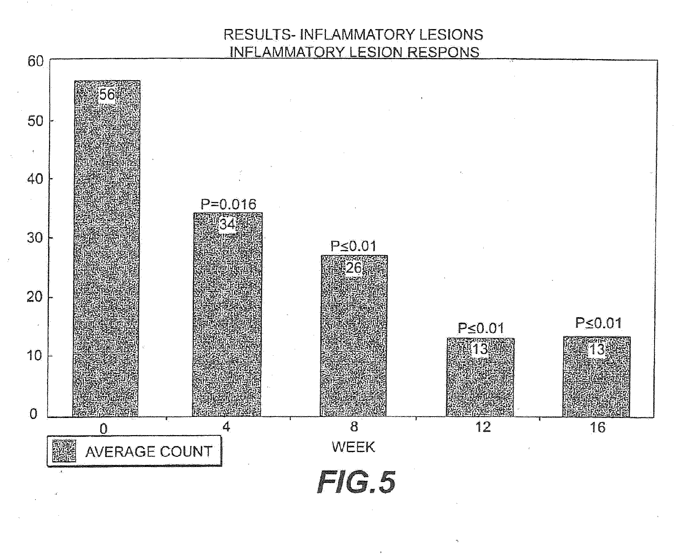

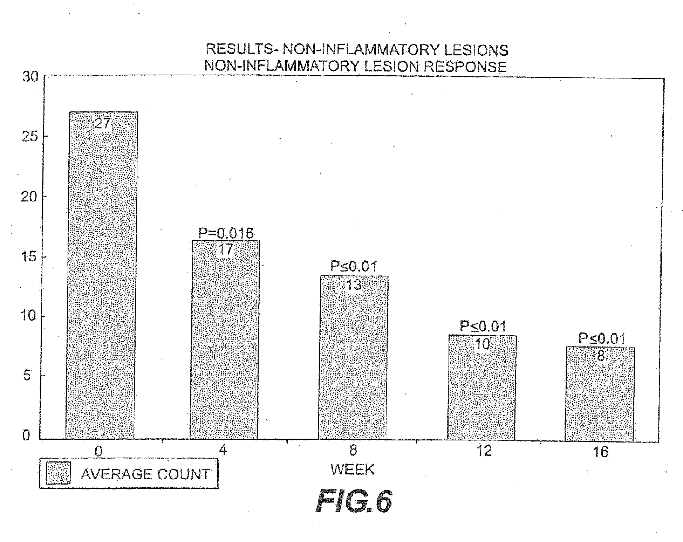

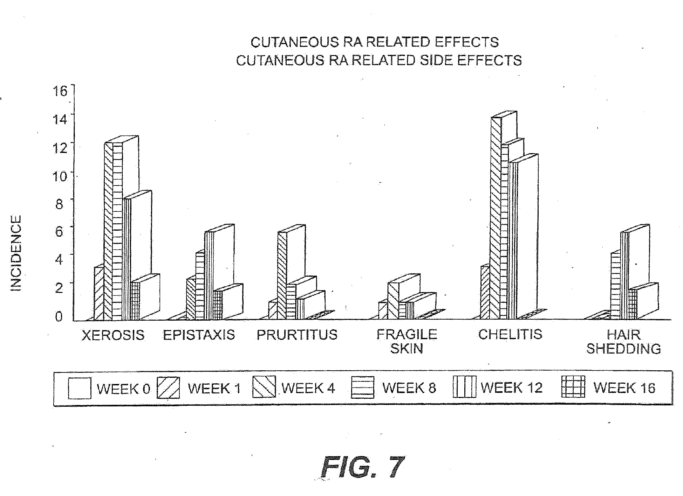

Sixteen patients with moderate to severe facial acne vulgaris received a daily oral dose of 1 mg oframbazole for 12 weeks. As indicated in Table 1, all patients (100%) in the study had a reduction in inflammatory lesions equal or superior to 50% after 12 weeks of treatment. The average lesion reduction was 79%. See Tables 3 through 7 for additional individual data. See Figures 5 and 6, respectively, for a comparison of the response to treatment of inflammatory lesions versus non-inflammatory lesions. No serious adverse effects were reported. See Figure 7.

**Table 1: Rambazole Treatment Resulting in ≥ 50% Reduction in Inflammatory Lesions**

| | **Week 4** | **Week 8** | **Week 12** | **Week 16 (follow-up)** |
|---|---|---|---|---|
| **No. of Subjects** | **16** | **16** | **16** | **16** |
| **No. of Responders** | **5** | **9** | **16** | **14** |
| **Percent Responders** | **31%** | **56%** | **100%** | **88%** |

### Example 3: Treatment of Acne Vulgaris with Topical Rambazole

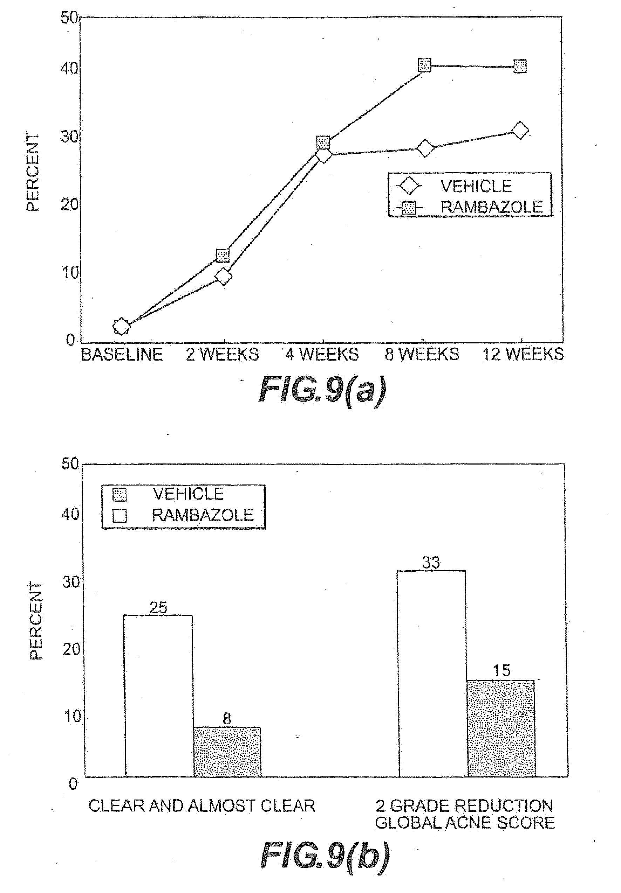

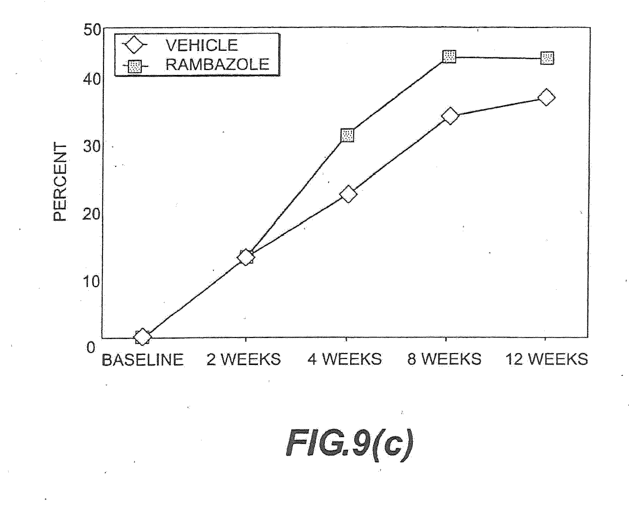

Fifty patients with mild to moderate acne vulgaris received a daily dose of topical 0.35% rambazole for 12 weeks. As indicated in Figure 9(a), patients in the study had a reduction in their global assessment score equal to approximately 40% after 12 weeks of treatment. Figure 9(b) shows a comparison of the success rate between rambazole and vehicle (i.e., the excipient components of the tested rambazole formulation). Figure 9(c) shows a comparison of the reduction of inflammatory lesions between rambazole and vehicle.

### Example 4: Effects of Topical 0.35% Rambazole on UVB

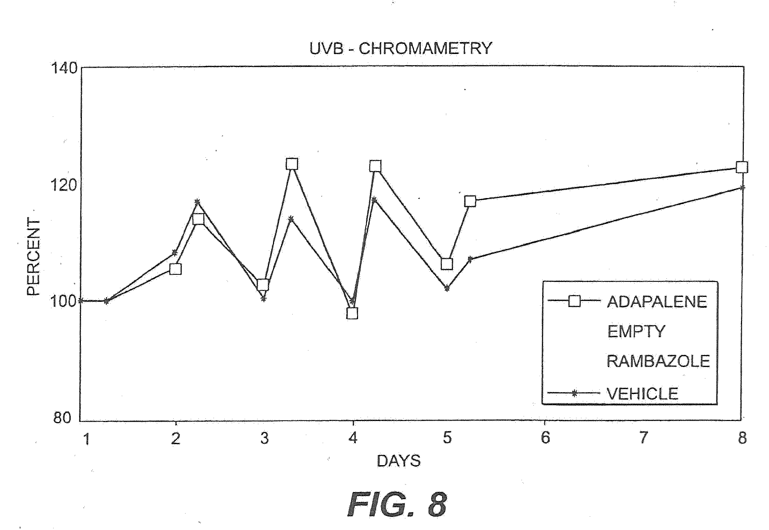

In an investigator-blinded, placebo-controlled study, topical 0.35% *rambazole* was applied to the skin of volunteers for 9 days, followed by 5 days of induction of inflammation with UVB. Adapalene was used in the study as a standard anti-inflammatory agent. Results as evaluated by chromametry showed no indication of phototoxicity. See Figure 8.

### Example 5: Effects of Topical 0.35% Rambazole on Irritant Dermatitis

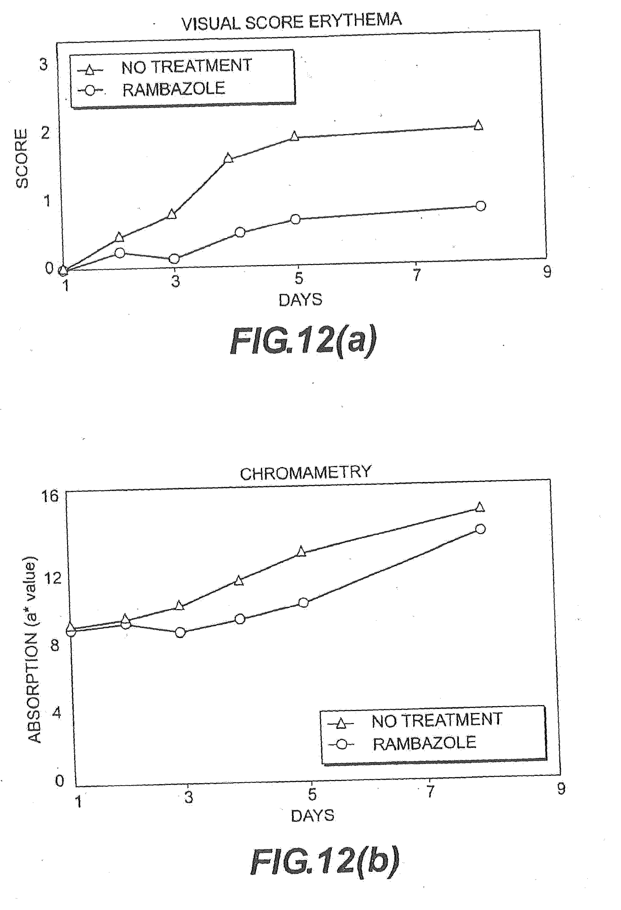

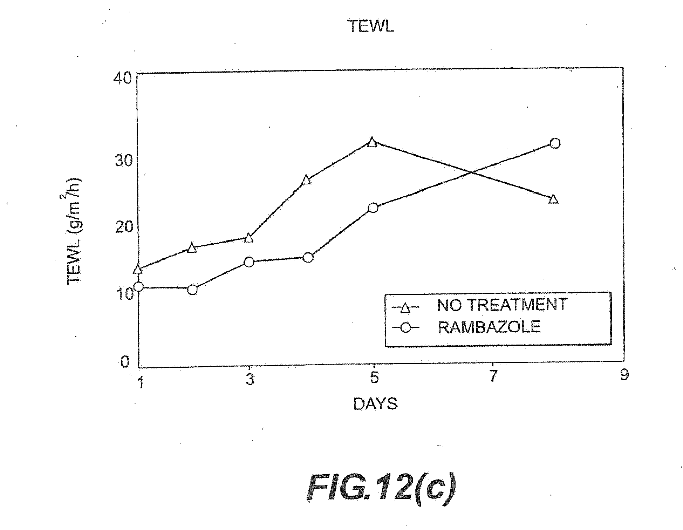

Rambazole (0.35%) gel was applied as a pre-treatment during four days. Inflammation was induced by applying 2% SLS during 2 hours in rings on the arm of twelve healthy volunteers once a day for five days. Rambazole (0.35%) gel was applied during the five days of induced inflammation. Skin reactions of non-treated and rambazole gel-treated skin sites were assessed 24 hours after each application with SLS and 72 hours after the last SLS application (visual scoring of redness, chromametry, TEWL). The results, depicted in Figures 12(a)-(c), show that rambazole gel inhibits the inflammatory response induced by application of SLS.

### Example 6: Effect of Rambazole on Levels of Interleukin 12 Secretion

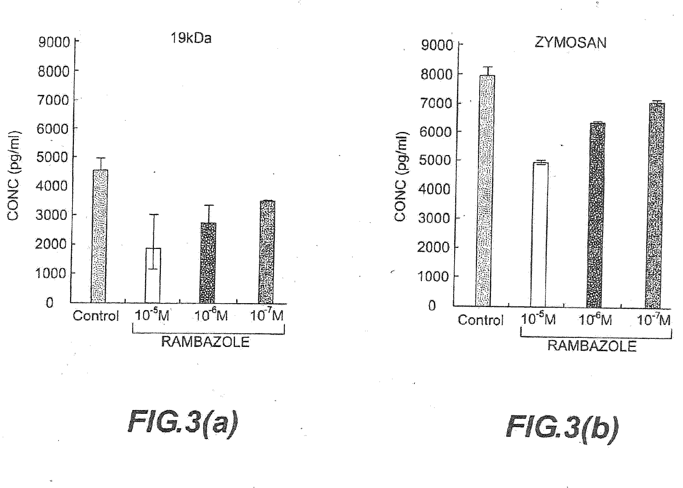

Peripheral blood mononuclear cells (PBMC) were isolated from healthy donors by density gradient centrifugation on Ficoll-Hypaque Plus and monocytes were selected by adherence. After overnight culture in RPMI 1640 supplemented with stimulated FBS, monocytes were activated with Zymosan or 19 kDa lipoprotein for 18h, and IL-12p40 was measured in the supernatant by ELISA. The results, depicted in Figure 3, show that rambazole dose-dependently suppressed IL-12 secretion.

### Example 7: Effect of Rambazole on Levels of IP-10 Secretion

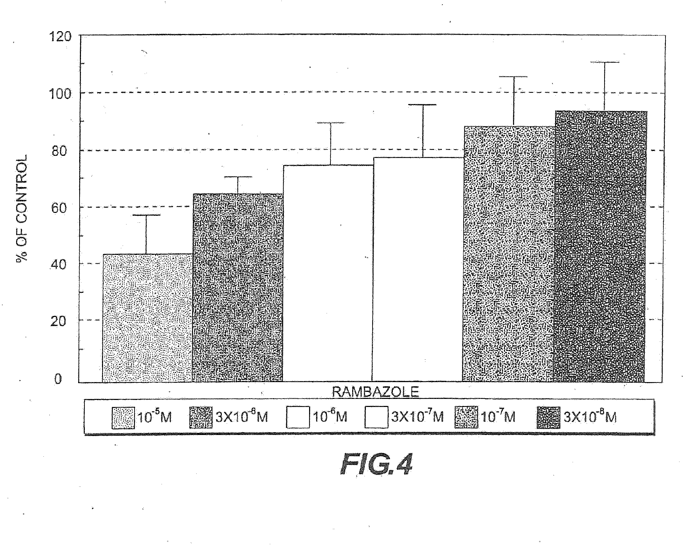

Fourth passage normal human epidermal keratinocytes were plated at 2,000 cells/well in low Ca²⁺ KSFM. After 3 days incubation to 60% confluence, the medium was removed and cells were stimulated with 50 U/mL IFNγ and incubated with rambazole ranging from 10-5 M to 3 x 10-8 M for 18 hours. Interferon-inducible protein (IP-10) expression in the culture medium was determined using the IP-10 sandwich-type ELISA and all data were corrected for cell count. Values are represented as mean percentages ± SD (n = 4) versus drug solvent control. The results, depicted in Figure 4, show that rambazole dose-dependently down regulated IP-10 expression and secretion.

### Example 8: Effects of Rambazole on the Expression of IL-1 in the Skin of Healthy Volunteers

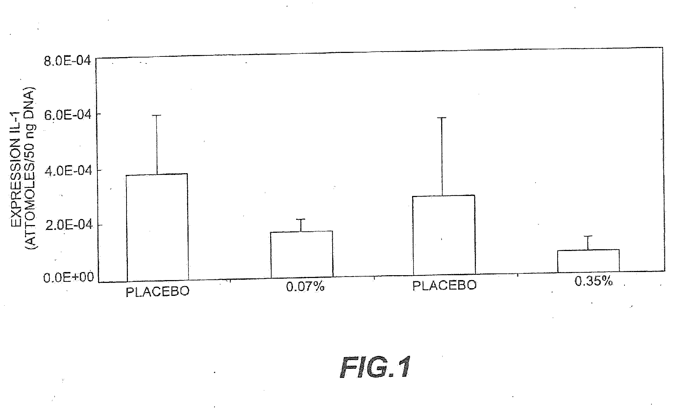

In this randomized, double-blind, left-right, placebo-controlled trial, the expression of Interleukin-1 (IL-1) in the skin of 15 healthy subjects was evaluated after a once daily application of rambazole gel at two different concentrations (0.35% or 0.07%) on intact skin for 9 days. The IL-1 biomarker was analyzed with real-time PCR. Treatment with rambazole was observed to dose-dependently decrease IL-1 expression. See Figure 1. During the 9 days of treatment, no itching, irritating, stinging, burning or vesiculation occurred, except for one subject who reported mild itching during one day after 8 days of treatment with 0.35% of rambazole.

Unless defined otherwise, all technical and scientific terms herein have the same meaning as commonly understood by one of ordinary skill in the art to which this invention belongs. Although any methods and materials, similar or equivalent to those described herein, can be used in the practice or testing of the present invention, the preferred methods and materials are described herein.

## Claims

1. A triazole compound selected from Formula I or a pharmaceutically acceptable salt thereof for use in a method of reducing or preventing inflammation in a mammal, 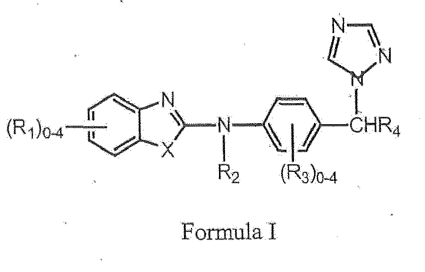 wherein
X is N, O or S;
each R₁ and each R₃ is independently selected from the group consisting of C₁₋₈ alkyl, amino, alkylamino, halogen, hydroxy, alkoxy, aryl, aryloxy, -CF₃, -OCF₃, -CORₐ, -COORₐ, - CONRₐR_{b}, -NHCORₐR_{b}, -NHSO₂Rₐ, -SO₂Rₐ, -SO₃Rₐ or -SO₂NRₐR_{b}, wherein Rₐ and R_{b} are independently hydrogen, alkyl, cycloalkyl, aryl or aralkyl;
R₂ is selected from the group consisting of H, C₁₋₆ alkyl, -CORₐ and -SO₂Rₐ; and
R₄ is selected from the group consisting af C₁₋₈ alkyl, optionally substituted by at least one group selected from the group consisting of amino, alkylamino, halogen, hydroxy, alkoxy, -CF₃, -OCF₃, -CORₐ, -COORₐ and -CONRₐR_{b}.

2. The compound for use according to claim 1, wherein the inflammation is caused by a disease state selected from the group consisting of inflammatory skin disease, arthritis, toxic shock syndrome, diabetes and inflammatory bowel disease.

3. The compound for use according to claim 2, wherein the inflammatory skin disease is selected from the group consisting of atopic dermatitis, contact dermatitis, seborrheic dermatitis, rosacea, eczema, psoriasis, acne and urticaria.

4. The compound for use according to claim 2, wherein the arthritis is selected from the group consisting of rheumatoid arthritis, psoriatic arthritis, osteoarthritis, gout, lupus and fibromyalgia.

5. The compound for use according to claim 2, wherein the inflammatory bowel disease is Crohn's disease.

6. The compound for use according to claim 2, wherein the diabetes is type 2.

7. The compound for use according to claim 1, wherein X = S and R₄ = a C₅ alkyl group.

8. The compound for use according to claim 1, wherein X = S, R₄ = a C₅ alkyl group and R₂ = H.

9. The compound for use according to claim 1, wherein X = S, R₄ = a C₅ alkyl group, R₂ = H and there are zero R₃ or R₁ groups.

10. The compound for use according to claim 1, wherein the triazole compound is rambazole.

11. The compound for use according to claim 1 or claim 10, wherein the triazole compound is administered orally or topically.

12. The compound for use according to claim 11, wherein the triazole compound is administered orally and wherein the amount of the triazole compound administered is in the range of:
0.05 mg to 1 g per day; or
0.5 mg to 500 mg per day; or
1 mg to 300 mg per day.

13. The compound for use according to claim 11, wherein the triazole compound is administered topically and wherein the concentration of the triazole compound administered is in the range of:
0.02 percent to 1 percent; or
0.05 percent to 0.80 percent.

14. The compound for use according to claim 13, wherein the concentration of the triazole compound administered is 0.07 percent, or 0.35 percent.

15. The compound for use according to claim 1, wherein the mammal is a human.

16. The compound for use according to claim 1, wherein the effective amount is the amount of the triazole compound necessary to decrease IL-1 or IL-12 expression and or activity.

## Patentansprüche

1. Eine Triazolverbindung ausgewählt aus Formel I oder ein pharmazeutisch verträgliches Salz davon zur Verwendung bei einem Verfahren zur Verringerung oder Vorbeugung einer Entzündung bei einem Säuger, 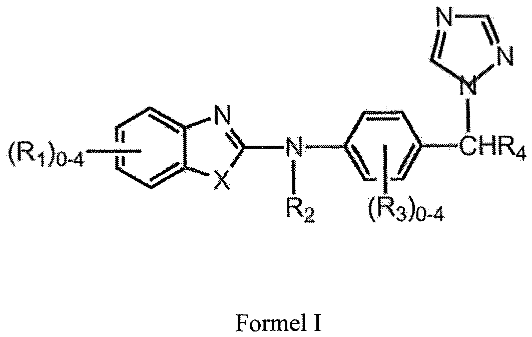 wobei
X gleich N, O oder S ist;
jedes R₁ und jedes R₃ unabhängig voneinander ausgewählt ist aus der Gruppe bestehend
aus C₁₋₈-Alkyl, Amino, Alkylamino, Halogen, Hydroxy, Alkoxy, Aryl, Aryloxy, -CF₃, -OCF₃, -CORₐ, -COORₐ, -CONRₐR_{b}, NHCORₐR_{b}, NHSO₂Rₐ, -SO₂Rₐ, -SO₃Rₐ oder -SO₂NRₐR_{b}, wobei Rₐ und R_{b} unabhängig Wasserstoff, Alkyl, Cycloalkyl, Aryl oder Aralkyl sind;
R₂ ausgewählt ist aus der Gruppe bestehend aus H, C₁₋₆-Alkyl, -CORₐ und -SO₂Rₐ; und
R₄ ausgewählt ist aus der Gruppe bestehend aus C₁₋₈-Alkyl, gegebenenfalls substituiert mit mindestens einem Rest ausgewählt aus der Gruppe bestehend aus Amino, Alkylamino, Halogen, Hydroxy, Alkoxy, -CF₃, -OCF₃, -CORₐ, -COORₐ und -CONRₐR_{b}.

2. Die Verbindung zur Verwendung gemäß Anspruch 1, wobei die Entzündung durch einen Krankheitszustand ausgewählt aus der Gruppe bestehend aus entzündlicher Hautkrankheit, Arthritis, toxischem Schocksyndrom, Diabetes und entzündlicher Darmerkrankung verursacht wird.

3. Die Verbindung zur Verwendung gemäß Anspruch 2, wobei die entzündliche Hauterkrankung ausgewählt ist aus der Gruppe bestehend aus atopischer Dermatitis, Kontaktdermatitis, seborrhoische Dermatitis, Rosazea, Ekzem, Psoriasis, Akne und Nesselfieber.

4. Die Verbindung zur Verwendung gemäß Anspruch 2, wobei die Arthritis ausgewählt ist aus der Gruppe bestehend aus rheumatoider Arthritis, psoriatischer Arthritis, Osteoarthritis, Gicht, Lupus und Fibromyalgie.

5. Die Verbindung zur Verwendung gemäß Anspruch 2, wobei die entzündliche Darmerkrankung Morbus Crohn ist.

6. Die Verbindung zur Verwendung gemäß Anspruch 2, wobei der Diabetes vom Typ 2 ist.

7. Die Verbindung zur Verwendung gemäß Anspruch 1, wobei X = S und R₄ = ein C₅-Alkylrest.

8. Die Verbindung zur Verwendung gemäß Anspruch 1, wobei X = S, R₄ = ein C₅-Alkylrest und R₂ = H.

9. Die Verbindung zur Verwendung gemäß Anspruch 1, wobei X = S, R₄ = ein C₅-Alkylrest, R₂ = H und keine R₃- oder R₁-Reste vorliegen.

10. Die Verbindung zur Verwendung gemäß Anspruch 1, wobei die Triazolverbindung Rambazol ist.

11. Die Verbindung zur Verwendung gemäß Anspruch 1 oder Anspruch 10, wobei die Triazolverbindung oral oder topisch verabreicht wird.

12. Die Verbindung zur Verwendung gemäß Anspruch 11, wobei die Triazolverbindung oral verabreicht wird und wobei die Menge der verabreichten Triazolverbindung im Bereich von:
0,05 mg bis 1 g pro Tag; oder
0,5 mg bis 500 mg pro Tag; oder
1 mg bis 300 mg pro Tag
liegt.

13. Die Verbindung zur Verwendung gemäß Anspruch 11, wobei die Triazolverbindung topisch verabreicht wird und wobei die Konzentration der verabreichten Triazolverbindung im Bereich von:
0,02 Prozent bis 1 Prozent; oder
0,05 Prozent bis 0,80 Prozent
liegt.

14. Die Verbindung zur Verwendung gemäß Anspruch 13, wobei die Konzentration der verabreichten Triazolverbindung 0,07 Prozent oder 0,35 Prozent beträgt.

15. Die Verbindung zur Verwendung gemäß Anspruch 1, wobei der Säuger ein Mensch ist.

16. Die Verbindung zur Verwendung gemäß Anspruch 1, wobei die wirksame Menge die Menge der Triazolverbindung ist, die nötig ist, um IL-1- oder IL-12-Expression und/oder -Aktivität zu verringern.

## Revendications

1. Dérivé de triazole choisi entre un composé de formule 1 et un de ses sels pharmaceutiquement acceptables, pour une utilisation dans un procédé de réduction ou de prévention de l'inflammation chez un mammifère, 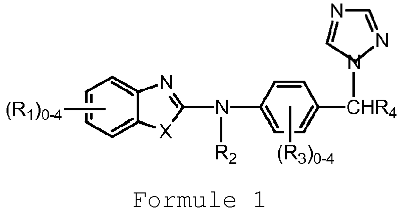 formule dans laquelle
X représente N, O ou S ;
chaque groupe R₁ et chaque groupe R₃ sont choisis indépendamment dans le groupe consistant en des groupes alkyle en C₁ à C₈, amino, alkylamino, halogéno, hydroxy, alkoxy, aryle, aryloxy, -CF₃, -OCF₃, -SORₐ, -COORₐ, -CONRₐR_{b}, -NHCORₐR_{b}, -NHSO₂Rₐ, -SO₂Rₐ, -SO₃Rₐ et SO₂NRₐR_{b}, dans lesquels Rₐ et R_{b} représentent indépendamment un atome d'hydrogène, un groupe alkyle, cycloalkyle, aryle ou aralkyle ;
R₂ est choisi dans le groupe consistant en H, des groupes alkyle en C₁ à C₆, -CORₐ et -SO₂Rₐ ; et
R₄ est choisi dans le groupe consistant en des groupes alkyle en C₁ à C₈, facultativement substitués avec au moins un groupe choisi dans le groupe consistant en des groupes amino, alkylamino, halogéno, hydroxy, alkoxy, -CF₃, -OCF₃, -CORₐ, -COORₐ et -CONRₐR_{b}.

2. Composé pour une utilisation suivant la revendication 1, l'inflammation étant provoquée par un état pathologique choisi dans le groupe consistant en une maladie cutanée inflammatoire, l'arthrite, le syndrome de choc toxique, le diabète et une maladie intestinale inflammatoire.

3. Composé pour une utilisation suivant la revendication 2, la maladie cutanée inflammatoire étant choisie dans le groupe consistant en la dermatite atopique, la dermatite de contact, la dermatite séborrhéique, la rosacée, l'eczéma, le psoriasis, l'acné et l'urticaire.

4. Composé pour une utilisation suivant la revendication 2, l'arthrite étant choisie dans le groupe consistant en la polyarthrite rhumatoïde, l'arthrite psoriasique, l'arthrose, la goutte, le lupus et la fibromyalgie.

5. Composé pour une utilisation suivant la revendication 2, la maladie intestinale inflammatoire étant la maladie de Crohn.

6. Composé pour une utilisation suivant la revendication 2, le diabète étant le diabète de type 2.

7. Composé pour une utilisation suivant la revendication 1, dans lequel X représente S et R₄ représente un groupe alkyle en C₅.

8. Composé pour une utilisation suivant la revendication 1, dans lequel X représente S, R₄ représente un groupe alkyle en C₅ et R₂ représente H.

9. Composé pour une utilisation suivant la revendication 1, dans lequel X représente S, R₄ représente un groupe alkyle en C₅, R₂ représente H et il existe 0 groupe R₃ ou R₁.

10. Composé pour une utilisation suivant la revendication 1, ledit dérivé de triazole étant le rambazole.

11. Composé pour une utilisation suivant la revendication 1 ou la revendication 10, ledit dérivé de triazole étant administré par voie orale ou topique.

12. Composé pour une utilisation suivant la revendication 11, ledit dérivé de triazole étant administré par voie orale et la quantité de dérivé de triazole administrée étant comprise dans l'intervalle de :
0,05 mg à 1 g par jour ; ou
0,5 mg à 500 mg par jour ; ou
1 mg à 300 mg par jour.

13. Composé pour une utilisation suivant la revendication 11, ledit dérivé de triazole étant administré par voie topique et la concentration du dérivé de triazole administrée étant comprise dans l'intervalle de :
0,02 % à 1 % ; ou
0,05 % à 0,80 %.

14. Composé pour une utilisation suivant la revendication 13, la concentration du dérivé de triazole administrée étant égale à 0,07 % ou 0,35 %.

15. Composé pour une utilisation suivant la revendication 1, le mammifère étant un être humain.

16. Composé pour une utilisation suivant la revendication 1, la quantité efficace étant la quantité du dérivé de triazole nécessaire pour diminuer l'expression et/ou l'activité de IL-1 ou IL-12.
